Europäisches Patentamt

European Patent Office

Office européen des brevets

(19)

(11) Publication number: **0 029 534**
**B1**

## EUROPEAN PATENT SPECIFICATION

(45) Date of publication of patent specification: **04.04.84**

(21) Application number: **80106850.3**

(22) Date of filing: **06.11.80**

(51) Int. Cl.³: **A 61 K 31/68**, A 61 K 9/14, A 61 K 9/20

(54) **Solid preparation comprising cobamamide or mecobalamin and process for preparing same.**

(30) Priority: **22.11.79 JP 150743/79**

(43) Date of publication of application:
**03.06.81 Bulletin 81/22**

(45) Publication of the grant of the patent:
**04.04.84 Bulletin 84/14**

(84) Designated Contracting States:
**BE DE FR GB IT**

(56) References cited:
**BE - A - 753 787**
**DE - A - 1 617 638**

**P.H. LIST et al.: "HAGERS HANDBUCH DER PHARMAZEUTISCHEN PRAXIS" vol.2, 1969, 4th edition, Springer Verlag BERLIN / HEIDELBERG / NEW YORK pages 34,35**

(73) Proprietor: **Eisai Co., Ltd.**
**6-10, Koishikawa 4-chome Bunkyo-ku Tokyo 112 (JP)**

(72) Inventor: **Iwagiri, Susumu**
**Takehayamachi Kawashimacho Hajima-gun, Gifu (JP)**
Inventor: **Hattori, Teiichi**
**11-26, AzaSagidera Goromaru Ohaza, Inuyama-shi Aichi (JP)**
Inventor: **Nasu, Teruyoshi**
**214, AzaKitaura Kotaiji-cho Tsushima-shi Aichi (JP)**

(74) Representative: **Hansen, Bernd, Dr.rer.nat. et al, Hoffmann, Eitle & Partner Patentanwälte Arabellastrasse 4 D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Solid preparation comprising cobamamide or mecobalamin and process for preparing same

This invention relates to a solid preparation comprising cobamamide or mecobalamin and a process for preparing same.

The cobamamide and mecobalamin both belong to coenzyme $B_{12}$. More particularly, the cobaamamide is a coenzyme $B_{12}$ occuring in liver, and the mecobalamin is a coenzyme $B_{12}$ occuring in blood. Both can be used as therapeutic agents for treating pernicious anemia. The mecobalamin is further effective as a therapeutic agent for treating peripheral nerve disease.

It is a known that coenzyme $B_{12}$ is extremely unstable to light. Accordingly, a variety of methods have been proposed for stabilizing coenzyme $B_{12}$. Examples are as follows:

To an aqueous coenzyme $B_{12}$ solution is added gelatin or dextran (Japanese patent publication (referred to, hereinafter as JPP) NO. 46(1971)—15320); a water-soluble coal tar dye (JPP No. 45(1970)—35798); a porphyrin compound, p-amino-benzoic acid or nicotonic amide (JPP NO. 45(1970)—11920); fumaric acid (JPP No. 45(1970)—38552); dextran-iron, sodium glutamate or sodium citrate (Japanese Provisional Patent Publication No. 49(1974)—418); ascorbic acid or eryssorbic acid (JPP No. 53(1978)—1810), or ethylene glycol or sorbitol (JPP No. 46(1971)—28093).

Coenzyme $B_{12}$ has been previously administered in most cases through injection. Accordingly the prior art cited above relates to stabilization of an aqueous solution of coenzyme $B_{12}$.

Recently, however, oral administration of the coenzymes $B_{12}$ has been increasingly adopted. Thus, the stabilization of coenzyme $B_{12}$ in solid preparations has now been under study. In view of the fact that cobamamide and mecobalamin incorporated into solid preparations are particularly unstable to light, the present inventors made studies for the purpose of providing a stabilzing means. As a result of the studies, it was found that cobamamide or mecobalamin contained in a solid preparation show high stability to light when present with a food red dye in the preparation. The present invention has been completed based on the above finding.

The Japanese Patent Publication No. 45(1970)—35798 describes that a coenzyme $B_{12}$-containing injection solution can be stabilized against light by the addition of a water-soluble coal tar dye. In examples 1 to 6, for instance, there is disclosed the addition of Evans' Blue, Methylene Blue, Indigo Carmine, phenolphthalein, methyl-rosaniline and Amaranth.

The Japanese Patent Publication No. 46(1971)—29740 describes that the aqueous methylcobalamin solution can be stabilized against light by the addition of a compound specifically adsorbing light in the range of 250

— 600 nm. In examples 1 to 10, for instance, there is disclosed the addition of Amaranth (Red Dye No. 2) and Sunset Yellow (Yellow Dye No. 5).

BE—A—753 787 describes preparations of vitamin $B_{12}$ in sugar sirup. In order to improve the stability of the vitamin $B_{12}$ complexes a dye-stuff is added, which absorbs light in the range of 200 to 600 nm.

Although the above-cited publications teach that a variety of compounds such as water-soluble coal tar and light-absorbing compound are useful as stabilizing agents for coenzyme $B_{12}$ contained in an aqueous solution, the studies carried out by the present inventors uncovered that concerning cobamamide and mecobalamin in a solid preparation, only the addition of a food red dye is effective for imparting stability to light to cobamamide and mecobalamin. A particularly excellent stabilizing effect is obtained with New Coccine (Red Dye No. 102).

As far as the solid prepartion is concerned, it is possible that the cobamamide or mecobalamin and the red dye can be individually formed in respective granules in advance of the admixing and then both are admixed to be present together. According to the studies, this individually granulating method can give the cobamamide or mecobalamin a stability at the same level as or higher than when cobamamide or mecobalamin and the red dye are admixed in powder, or when both are admixed into a single granule. Thus a preferable embodiment of the invention is the solid preparation in which cobamamide or mecobalamin and the red coloring matter are present separately in different granules.

The present invention for stabilizing a solid preparation of cobamamide and mecobalamin is therefore believed to be unobvious from the pior art disclosing the stabilization of an aqueous co-enzyme $B_{12}$ preparation.

Red dyes to be used in the present invention include Amaranth (Red Dye No. 2), Erythrocin (Red Dye No. 3), New Coccine (Red Dye No. 102), Phloxine (Red Dye No. 104), Rose Bengale (Red Dye No. 105) and Acid Red (Red Dye No. 106). Particularly, New Coccine provides excellent appearance to the solid preparation as well as the extraordinary stabilization as shown hereinafter in the examples.

The amount of the red dye to be added lies in the range of 0.1 to 5 parts of the red dye, based on one part of cobamamide or mecobalamin. This range can be similarly applied to either when cobamamide or mecobalamin and the red dye are directly admixed in powdery form or when both are admixed after each granulation step.

Thus the invention relates to a solid preparation comprising cobamamide or mecobalamin and a pharmaceutical solid carrier, characterized in that the cobamamide or mecobalamin is present together with a red dye selected from the group Amaranth, Erythrocin, New Coccine, Phloxine, Rose Bengale and/or Acid Red in an amount from 0.1 to 5 parts by weight, based on one part by weight of said cobamamide or mecobalamin.

When cobamamide or mecobalamin and the red dye are individually granulated prior to admixing, the ratio between both granules can vary optionally with the desired amount of cobamamide or mecobalamin. If the mecobalamin content, for instance, should be 0.1%, granules containing 1% of mecobalamin are prepared in the first place, and then the granules are diluted ten times with the separately prepared granules containing the red dye. That is to say, both are admixed in the ratio of 1 : 9.

Examples of the granules of the present invention include granules prepared by the cylindrical granulating method, as well as other, such as fine granules prepared by the fluidized-bed granulating method. Accordingly, the granules to be employed for the individually (separatingly) granulating process can be optionally prepared by any of the conventional methods such as the cylindrical granulating method, the tumbling granulating method, the fluidized-bed granulating method, etc.

Representative examples of the solid preparations of the present invention include a powder, a granule, a capsule and a tablet. Therefore the cobamamide or mecobalamin and the red dye can be together present in the solid preparation, in the first place, in one of four states such as (1) a state in which both are present in a powdery preparation; (2) a state in which both are together present in the same granule; (3) a state in which one is present in the granular form while the other, in the powder form; and (4) a state in which both are present in the individually granulated form. Furthermore, these powders and granules can be encapsulated or tableted.

A pharmaceutical composition according to the invention, as shown above, comprises cobamamide or mecobalamin, a red dye suitable for food and a carrier. As the carrier, there may be used an conventional ones, for example, corn starch, lactose, mannitol, crystalline cellulose and hydroxypropyl cellulose.

Brief Description of the Drawings

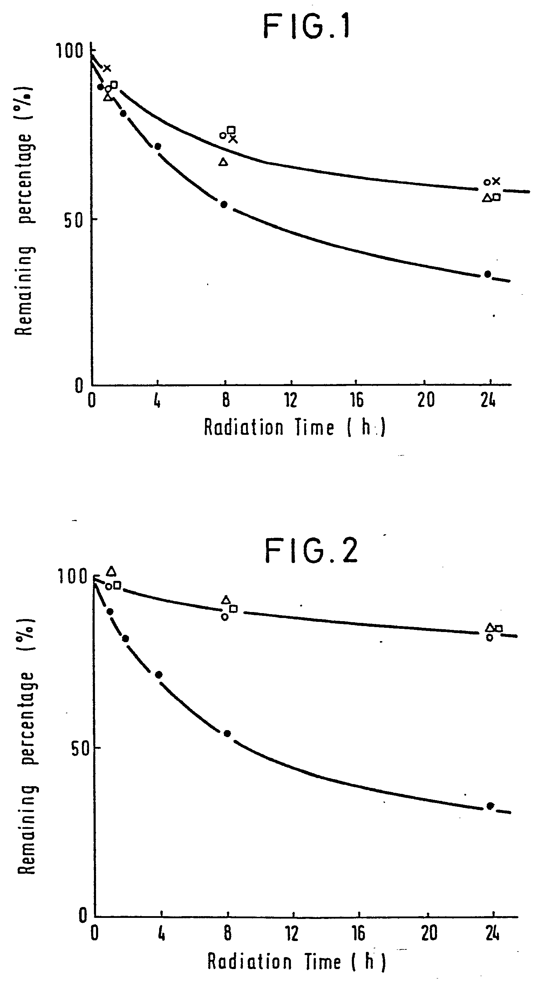

Figure 1 shows the change of percentages of the remaining mecobalamin with the passage of time under conditions in which mecobalamin and New Coccine were included in the same granule and 1,000 Lux of light was radiated onto the granules. In the figure, the lines indicate the cases in which the New Coccine contents were 0% (●), 0.1% (o), 0.2% (△), 0.4% (□) and 0.6% (x), respectively.

Figure 2 shows the change of remaining mecobalamin percentage with the passage of time under conditions which the mecobalamin and New Coccine were included in different granules and 1,000 Lux of light was radiated onto the granules. In the figure, the lines indicate the cases in which the New Coccine contents were 0.1% (o), 0.2% (△), and 0.4 (□), respectively. The control specimen is indicated by the line marked (●).

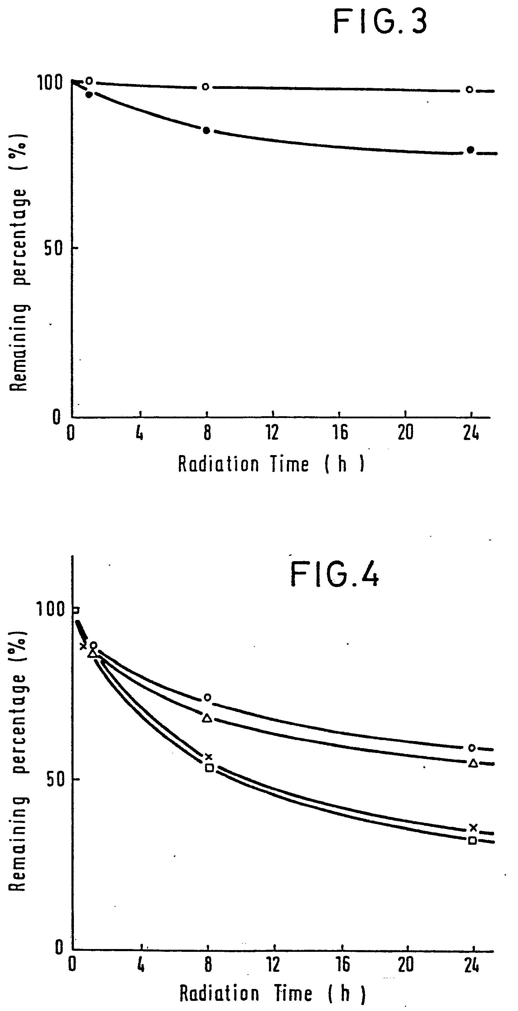

Figure 3 shows the change of remaining cobamamide percentages with the passage of time under conditions in which the cobamamide and New Coccine were included in different granules and 1,000 Lux of light was radiated onto the granules. In the figure, the lines indicate the case in which the New Coccine content was 0.2% (o), and the control specimen was (●), respectively.

Figure 4 shows the change of remaining mecobalamin percentages with the passage of time under conditions in which the mecobalamin and one of the food dyes were included in the same graunule and 1,000 Lux of light was radiated onto the granules. In the figure, the lines indicate the cases in which the food dyes were New Coccine (o), Erythrocin (△), Tartrazine (x), and Indigo Carmine (□).

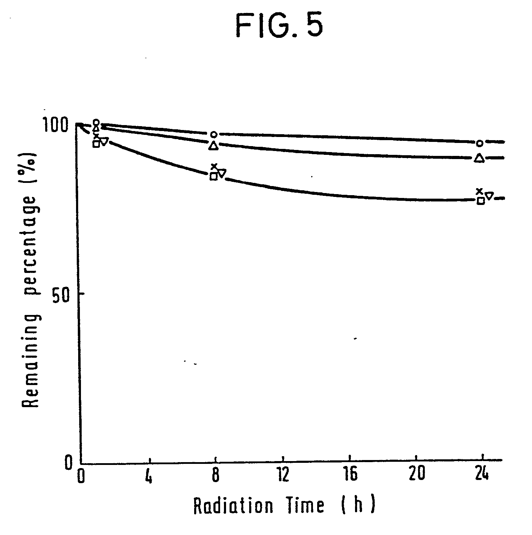

Figure 5 shows the change of remaining cobamamide percentages with the passage of time under conditions in which the cobamamide and one of the fodd dyes were included in the same granule and 1,000 Lux of light was radiated onto the granules. In the figure, the lines indicate the cases in which the food dyes were New Coccine (o), Rose Bengale (△), Sunset Yellow (x), Fast Green (□), and Brilliant Blue (▽).

The effect given by the present invention is shown below by respective examples.

Example of Effect 1

*Specimen*

Granules were prepared in the same manner as in the procedure described in Example 1 (given hereinafter). Four kinds of specimens in which the New Coccine contents were 0.1%, 0.2%, 0.4% and 0.6%, respectively, were prepared. In the specimens, mecobalamin and New Coccine were together present in the same granule. A control specimen was prepared in the same manner as above, except that no New Coccine was included therein.

*Test Method*

In a Petri dish (diameter: 9 cm) were placed 4 g of the specimen evenly so that the height of the specimn layer reached 1 — 2 mm. The so spread specimen was left under a fluorescent lamp by 1,000 Lux. Percentages of remaining mecobalamin were determined at regular intervals.

*Results*

The results are illustrated in Figure 1. It is evident according to Figure 1 that New Coccine is effective in stabilizing mecobalamin against light.

Example of Effect 2

*Specimen*

Granules were prepared in the same manner as in the procedure described in Example 2 (given hereinafter); three kinds of specimens in which the New Coccine contents were 0.1%, 0.2% and 0.4%, respectively. In each specimens, mecobalamin and New Coccine had been individually granulated prior to the admixing. A control specimen employed was the same as that employed in Example of Effect 1.

*Test Method*

The same method as described in Example of Effect 1.

*Results*

The results are illustrated in Figure 2. It is evident according to Figure 2 that New Coccine gives higher light-stability to mecobalamin when New Coccine and mecobalamin are individually granulated in advance of the admixing into the preparation.

Example of Effect 3

*Specimen*

Granules were prepared in the same manner as in the procedure described in Example 3 (given hereinafter); the New Coccine content was adjusted to 0.2%. In the specimen, cobamamide and New Coccine had been individually granulated prior to the admixing. A control specimen was prepared in the same manner as described in Example 5 (given hereinafter) except that no New Coccine was included therein.

*Test Method*

The same method as described in Example of Effect 1.

*Results*

The results are illustrated in Figure 3. It is evident according to Figure 3 that New Coccine gives higer light-stability to cobamamide when New Coccine and cobamamide are individually granulated in advance of the admixing into the preparation.

Example of Effect 4

*Specimen*

Capsules were prepared in the same manner as in the procedure described in Example 4 (given hereinafter); two kinds of specimens were prepared in which the New Coccine contents per one capsule was 0 mg and 0.1 mg, respectively.

*Test Method*

The specimens were placed in a Petri dishes and kept under two conditions, that is, 8 h x one month under 1,000 Lux (240,000 Lux.h) and 8 h x three months under 1,000 Lux (720,000 Lux.h). Then, percentages of the remaining portion were determined.

*Results*

The results are set forth in the following table. According to the results, it is evident that New Coccine works well in the capsule preparation for imparting the stabilization to mecobalamin.

| New Coccine Content per one capsule | 240,000 Lux.h | 720,000 Lux.h |
|---|---|---|
| 0 mg | 86.3% | 83.8% |
| 0.1 mg | 95.7% | 95.0% |

Example of Effect 5

*Specimen*

Granules were prepared in the same manner as in the procedure described in Example 1 (given hereinafter); four kinds of specimen were employed, containing as the dye New Coccine (Red Dye No. 102), Erythrocin (Red Dye No. 3), Tartrazine (Yellow Dye No. 4) and Indigo Carmine (Blue Dye No. 2), respectively.

*Test Method*

The same method as described in Example of Effect 1

*Results*

The results are illustrated in Figure 4. According to Figure 4, it is evident that the red dyes are specifically effective in rendering light-stabilization to mecobalamin.

Example of Effect 6

*Specimen*

Granules were prepared in the same manner as in the procedure described in Example 5 (given hereinafter); five kinds or specimens, each containing as the dye New Coccine (Red Dye No. 102), Rose Bengale (Red Dye No. 105), Sunset Yellow (Yellow Dye No. 5), Fast Green (Green Dye No. 3) or Brilliant Blue (Blue Dye No. 1).

*Test Method*

The same method as described in Example of Effect 1.

*Results*

The results are illustrated in Figure 5. According to Figure 5, it is evident that the red dyes are specifically effective in rendering light-stabilization to cobamamide.

The present invention are further illustrated by the following examples.

### Example 1

0.1 g of mecobalamin, 0.1 g of New Coccine and 98 g of mannitol were mixed, and the mixture was then kneaded with 20 ml of a 10% ethanolic solution of hydroxypropyl cellulose. This was granulated by means of a cylinder using a screen of the mesh 0.5 mm and dried for 10 h by blowing air maintained at about 60°C. The dried granular product was then dressed to give a product in the grain range of 1410 — 500 $\theta$m.

The thus obtained granule had a mecobalamin content of 0.1%. The New Coccine content therein was 0.1%.

### Example 2

1 g of mecobalamin 7 g of corn starch and 90 g of mannitol were mixed, and 40 ml of a 5% aqueous solution of hydroxypropyl cellulose was sprayed over the mixture. The resulting mixture was granulated and dried by the fluidized bed method. The dried granular product was then dressed to give a product in the grain range of about 500 — 105 $\theta$m. This granule was reffered to as Granule A.

Independently, 0.11 g of New Coccine, 7 g of corn starch and 91 g of mannitol were mixed, and 40 ml of an 5% aqueous solution of hydroxypropyl cellulose was sprayed over the mixture. The resulting mixture was then granulated and dried by the fluidized bed method. The dried granular product was subsequently dressed to give a product in the grain range of about 500 — 105 $\theta$m. This granule was referred to as Granule B.

Granule A and Granule B were mixed in the ratio of 1 : 9. The thus obtained granule had a mecobalamin content of 0.1%. The New Coccine content was 0.1%.

### Example 3

1 g of cobamamide, 7 g of corn starch and 90 g of mannitol were mixed, and the mixture was then kneaded with 20 ml of a 10% ethanolic solution of hydroxypropyl cellulose. This was granulated by means of a cylinder using a screen of the mesh 0.5 mm and dried for 10 h by blowing air maintained at about 60°C. The dried granular product was then dressed to give a product in the grain range of 1410 — 500 $\theta$m. This granule was referred to as Granule A.

Independently, 0.22 g of New Coccine, 7 g of corn starch and 91 g of mannitol were mixed, and the mixture was then kneaded with 20 ml of a 10% ethanol solution of hydroxypropanol cellulose. This was granulated by means of a cylinder using a screen of the mesh 0.5 mm and dried for 10 h by blowing air maintained at about 60°C. The dried granular product was then dressed to give a product in the grain range of 1410 — 500 $\theta$m. This granule was referred to as Granule B.

Granule A and Granule B were mixed in the ratio of 1 : 9. The thus obtained granule had a cobamamide content of 0.1%. The New Coccine content was 0.2%.

### Example 4

0.2 g of New Coccine, 100 g of crystalline cellulose and 100 g of lactose were mixed and kneaded to turn into a wet lump with red color, and the resulting lump was dried in the fluidized-bed drier. The dried granular product was subsequently dressed by means of a sieve of the mesh 841 $\theta$m. The resulting red-colored powder (100 g) was mixed with 0.25 g of mecobalamin, and the mixture was incorporated into No. 4 capsules with red-colored caps and white-colored bodies in the amount of 100.25 mg per a capsule.

The thus obtained capsule contained 0.25 mg of mecobalamin and 0.1 mg of New Coccine per each capsule.

### Example 5

0.1 g of cobamamide, 0.1 g. of New Coccine and 98 g of mannitol were mixed, and the mixture was then kneaded with 20 ml of a 10% ethanolic solution of hydroxypropyl cellulose. This was granulated by means of a cylinder using a screen of the mesh 0.5 mm and dried for 10 h by blowing air maintained at about 60°C. The dried granular product was then dressed to give a product in the grain range of 1410 — 500 $\theta$m.

The thus obtained granule had a cobamamide content of 0.1%. The New Coccine content was 0.1%.

### Claims

1. A solid preparation comprising cobamamide or mecobalamin and a pharmaceutical solid carrier, characterized in that the cobamamide or mecobalamin is present together with a red dye selected from the group Amaranth, Erythrocin, New Coccine, Phloxine, Rose Bengale and/or Acid Red in an amount from 0.1 to 5 parts by weight, based on one part by weight of said cobamamide or mecobalamin.

2. A solid preparation according to claim 1, in which the solid preparation is the form of powder, granule, capsule or tablet.

3. A solid preparation according to claim 1, in which the cobamamide or mecobalamin and the red dye are present individually in different granules.

4. A process for producing a solid pharmaceutical composition according to claim 1, which comprises the steps of: admixing combamamide or mecobalamin, the red dye and a pharmaceutical solid carrier and then forming the resulting mixture into a form of solid preparation.

5. A process according to claim 4, which comprises the steps of: mixing cobamamide or mecobalamin and a pharmaceutical solid carrier and separately mixing the red dye and a pharmaceutical solid carrier, then granulating the obtained two mixtures separately and combining the resulting two kinds of granulates into a form of solid preparation.

## Revendications

1. Une composition solide contenant de la cobamamide ou de la mécobalamine et un véhicule solide pharmaceutique, caractérisée en ce que la cobamamide ou la mécobalamine est présente avec un colorant rouge choisi dans le groupe formé par l'amaranthe, lérythrocine, le colorant New Coccine, la phloxine, le rose Bengale et/ou le rouge acide en quantité de 0,1 à 5 parties en poids pour 1 partie en poids de cobamamide ou de mécobalamine.

2. Une composition solide selon la revendication 1, à l'état de poudre, de granulés, de capsules ou de comprimés.

3. Une composition solide selon la revendication 1, dans laquelle la cobamamide ou la mécobalamine et le colorant rouge sont présents individuellement dans des granulés différents.

4. Un procédé pour préparer une composition pharmaceutique solide selon la revendication 1, qui comprend les stades suivants: on mélange la cobamamide ou la mécobalamine, le colorant rouge et un véhicule pharmaceutique solide, puis on met le mélange obtenu sous la forme d'une composition solide.

5. Un procédé selon la revendication 4 qui comprend les stades suivants: on mélange la cobamamide ou la mécobalamine et un véhicule pharmaceutique solide et on mélange séparément un colorant rouge et un véhicule pharmaceutique solide, on met les deux mélanges séparément à l'état de granulés puis on combine les deux types de granulés sous forme d'une composition solide.

## Patentansprüche

1. Festes Präparat, das Cobamamid oder Mecobalamin und einen pharmazeutischen festen Träger enthält, dadurch gekennzeichnet, dass das Cobamamid oder Mecobalamin zusammen mit einem roten Farbstoff aus der Gruppe Amaranth, Erythrocin, New Coccin, Phloxin, Rose Bengale und/oder Acid Red in einer Menge von 0,1 bis 5 Gewichtsteilen, bezogen auf einen Gewichtsteil des genannten Cobamamids oder Mecobalamins, vorliegt.

2. Festes Präparat gemäss Anspruch 1, dadurch gekennzeichnet, dass das feste Präparat in Form eines Pulvers, Granalien, Kapseln oder Tabletten vorliegt.

3. Festes Präpatat gemäss Anspruch 1, dadurch gekennzeichnet, dass das Cobamamid oder Mecobalamin und der rote Farbstoff individuell in verschiedenen Granalien vorliegen.

4. Verfahren zur Herstellung einer festen pharmazeutischen Zubereitung gemäss Anspruch 1, gekennzeichnet durch die folgenden Schritte: Vermischen des Cobamamids oder Mecobalamins, des roten Farbstoffes und eines pharmazeutischen festen Trägers und Konfektionieren des erhaltenen Gemisches in die Form eines festen Präparates.

5. Verfahren gemäss Anspruch 4, gekennzeichnet durch die folgenden Schritte: Vermischen des Cobamamids oder Mecobalamins und eines pharmazeutischen festen Trägers und getrenntes Vermischen des roten Farbstoffes und eines pharmazeutischen festen Trägers, anschliessendes Granulieren der erhaltenen zwei Gemische in getrennter Weise und Kombinieren der erhalten zwei Arten von Granulaten in eine Form eines festen Präparates.

# FIG.1

Remaining percentage (%)

Radiation Time ( h )

# FIG.2

Remaining percentage (%)

Radiation Time ( h )

0 029 534

# FIG.3

Remaining percentage ( % ) vs Radiation Time ( h )

# FIG.4

Remaining percentage (%) vs Radiation Time ( h )

2

# FIG. 5